Europäisches Patentamt

European Patent Office

Office européen des brevets

(11) Veröffentlichungsnummer: **0 017 966**
A1

# EUROPÄISCHE PATENTANMELDUNG

(21) Anmeldenummer: 80102028.0

(22) Anmeldetag 15.04.80

(51) Int. Cl.³ **A 61 B 5/04**

(30) Priorität 20.04.79 DE 2916067

(43) Veröffentlichungstag der Anmeldung **29.10.80**
**Patentblatt 80/22**

(84) Benannte Vertragsstaaten **AT BE CH FR GB IT LI NL SE**

(71) Anmelder: **SIEMENS AKTIENGESELLSCHAFT Berlin und München, Postfach 22 02 61, D-8000 München 22 (DE)**

(72) Erfinder: **Weigert, Kurt, Grillparzerstrasse 26, D-8510 Fürth (DE)**
Erfinder: **Molz, Claudius, Isarstrasse 4, D-8520 Erlangen (DE)**

(54) Verfahren und Vorrichtung zur Auswertung von im wesentlichen periodischen Signalfolgen.

(57) Die Erfindung bezieht sich auf ein Verfahren zur Auswertung von im wesentlichen periodischen Signalfolgen, insbesondere von physiologischen Signalen, wie EKG od.dgl., sowie auf eine Vorrichtung zur Durchführung dieses Verfahrens. Bei der automatischen EKG-Auswertung wird in einstellbaren Zeitintervallen das Auftreten von Signalen, wie regulärer QRS-Komplex oder auch Extrasystolen, überwacht. Es kommt jedoch auch vor, daß reguläre Signalkomplexe in einer Signalfolge fehlen. Gemäß der Erfindung wird zur Erkennung und Anzeige von in der im wesentlichen periodischen Signalfolge entsprechend mittlerer Signalfrequenz fehlenden Einzelereignissen eine justierbare, der Signalperiodendauer proportionale Meßspannung mit dem Mittelwert aus der in der vorangehenden Signalperiode aufsummierten Meßspannung verglichen, wobei bei Überschreiten des Vergleichsspannungswertes ein Zählsignal ausgelöst wird. Es können so Asystolien oder sog. «missed beat» des EKG's festgestellt werden, wobei gleichzeitig kompensatorische Pausen nach Extrasystolen eliminiert werden.

SIEMENS AKTIENGESELLSCHAFT     Unsere Zeichen
Berlin und München             VPA 79 P 5036  EUR


Verfahren und Vorrichtung zur Auswertung von im
wesentlichen periodischen Signalfolgen


Die Erfindung bezieht sich auf ein Verfahren zur Auswertung von im wesentlichen periodischen Signalfolgen, insbesondere von physiologischen Signalen, wie EKG od.dgl., sowie auf eine Vorrichtung zur Durchführung dieses Verfahrens.

Bei Auswertung von im wesentlichen periodischen Signalfolgen werden im allgemeinen signifikante Signalereignisse zur Erzeugung von Triggersignalen herangezogen. Diese werden dann hinsichtlich Periode bzw. Frequenz der signifikanten Ereignisse ausgewertet. Daneben interessiert im allgemeinen auch das Auftreten von außerordentlichen Ereignissen in der periodischen Folge von Normalereignissen, da diese insbesondere unmittelbar das Frequenzmeßergebnis beeinflussen.


Wht 5 Rl / 18.4.1979

Insbesondere bei Auswertung von physiologischen Signalkurven, beispielsweise einem Elektrokardiogramm, tritt neben der Frequenzmessung anhand der signifikanten Signalereignisse auch die eigentliche Strukturanalyse der Signale in den Vordergrund. Aus dem zeitlichen Verlauf des Auftretens von Extrasystolen kann beispielsweise der Mediziner diagnostische Aussagen ableiten. Beim Stand der Technik ist es daher üblich, den Abstand zwischen den normalen QRS-Komplexen auf Extrasystolen zu überwachen. Bekannte elektromedizinische Geräte arbeiten mit konstant vorgebbaren Zeitintervallen, in denen das Auftreten von Extrasystolen im EKG erfaßt und klassifiziert wird.

Auch aus dem Wegfall eines Normalereignisses in einer im wesentlichen periodischen Signalfolge können Aussagen abgeleitet werden. Speziell in der EKG-Auswertetechnik spricht man bei Fehlen eines normalen QRS-Komplexes von einer sog. Asystolie oder "missed beat". Derartige Asystolien oder "missed beat" können zwar bei EKG-Registrierung auf dem Lichtschirm eines Oszilloskops oder dem Registrierstreifen eines Kardiographen vom Mediziner sofort als Anomalien erkannt werden; bei der automatischen Auswertung von EKG-Kurven werden sie allerdings nicht ohne weiteres als solche erkannt und bewertet.

Der Erfindung liegt daher die Aufgabe zugrunde, ein Verfahren und eine Vorrichtung anzugeben, mit denen die automatische Auswertung von im wesentlichen periodischen Signalfolgen verbessert wird. Insbesondere für die EKG-Auswertetechnik soll die Möglichkeit geschaffen werden, vereinzelt auftretende Asystolien oder den Ausfall einzelner Schläge unabhängig von der mittleren Herzfrequenz zu registrieren.

Die Aufgabe wird erfindungsgemäß dadurch gelöst, daß
zur Erkennung und Anzeige von in der im wesentlichen
periodischen Signalfolge entsprechend mittlerer Herzfrequenz fehlende Einzelereignissen eine justierbare,
der Signalperiodendauer proportionale Meßspannung mit
dem Mittelwert aus der in der vorangehenden Signalperiode aufsummierten Meßspannung verglichen wird, wobei bei Überschreiten des Vergleichsspannungswertes
ein Zählimpuls ausgelöst wird.

Beim Verfahren nach der Erfindung wird also durch die
vorangehende Signalperiode ein mitlaufender Vergleichsspannungswert  definiert. Die in der laufenden Signalperiode ansteigende zeitproportionale Meßspannung kann
dann so justiert werden, daß erst bei Überschreiten
eines den mittleren Ereignisabstand um einen bestimmten
Multiplikationsfaktor übertreffenden Abstandes ein
Zählimpuls getriggert wird. Damit ist bei der EKG-Auswertung eine Asystolie oder ein "missed beat" unmittelbar anzeigbar. In vorteilhafter Ausführung wird die
Meßspannung daneben weiterhin mit einer einstellbaren
Konstantspannung verglichen, so daß auch bei Überschreiten eines maximalen vorgebbaren Zeitintervalls das Zählsignal erzeugt wird. Dieses Zeitintervall beträgt vorzugsweise 2 s, da bei einem EKG ein RR-Abstand größer
als 2 s in jedem Fall als pathologisch zu werten ist.

In Weiterbildung der Erfindung lassen sich auch solche
Ereignispausen, die lediglich aufgrund eines vorzeitig
auftretenden signifikanten Ereignisses verursacht werden, durch Vergleich der Meßspannung mit dem um den
Grad der Vorzeitigkeit entsprechend angehobenen Mittelwert der Vergleichsspannung erkennen. Bei der EKG-Auswertung lassen sich demnach kompensatorische Pausen
aufgrund von vorzeitig auftretenden Extrasystolen von
echten Asystolien oder "missed beat" unterscheiden.

0017966

- 4 -     VPA 79 P 5036 EUR

Eine Vorrichtung zur Durchführung des erfindungsgemäßen Verfahrens weist in seiner Schaltungsanordnung
einen Meßspannungssummierer, einen Mittelwertbildner,
einen Meßspannungsspeicher und wenigstens einen Komparator auf. Vorzugsweise sind in der praktischen Ausführung zur automatischen EKG-Auswertung jedoch drei
Komparatoren vorhanden, so daß die Anzeige einer
Asystolie oder "missed beat" bei Überschreiten eines
um einen vorbestimmbaren Faktor überschreitenden mittleren RR-Abstandes bei einem vorgegebenen Maximalintervall möglich ist und gleichzeitig kompensatorische
Pausen nach Extrasystolen erkannt werden. Dabei wird
die periodenproportionale Meßspannung in an sich bekannter Weise durch einen von einer Konstantstromquelle
aufladbaren Kondensator als Meßspannungssummierer gebildet, der jeweils bei Auftreten eines signifikanten
Signalereignisses entladen wird. Es wird so eine Sägezahnspannung entsprechend den Signalabständen erzeugt.
Die Höhe dieser Sägezahnspannung ist aber über Spannungsteiler entsprechend einem vorgebbaren Multiplikationsfaktor für einen mittleren Ereignisabstand justierbar.

Weitere Vorteile und Einzelheiten der Erfindung ergeben sich aus nachfolgender Figurenbeschreibung eines
Ausführungsbeispieles anhand der Zeichnung. Es zeigen:

Fig. 1 eine erfindungsgemäße Schaltungsanordnung und

Fig. 2 Impulsdiagramme der an den Schaltungspunkten
auftretenden Spannungen.

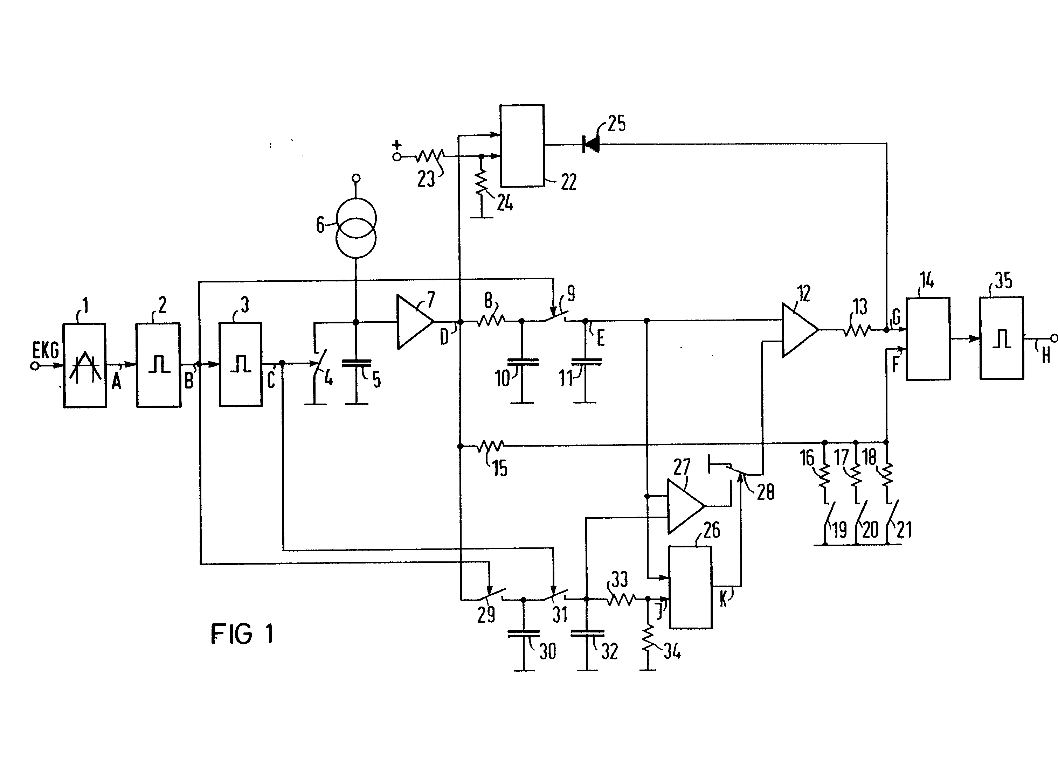

In der Fig. 1 wird einer Schaltungsanordnung ein EKG
eingespeist. Die Schaltungsanordnung besteht aus einer
Einheit 1 zur QRS-Erkennung, mit der bei  eine bestimmte Schwelle und Signalbreite überschreitenden QRS-Komplexen Rechteckimpulse gebildet werden. Der Einheit 1 sind zwei monostabile Multivibratoren (Monoflop) 2 und 3 nachgeschaltet, die entsprechend definierte Triggerimpulse erzeugen. In der Schaltung wird ein Kondensator 5 geeigneter Kapazität von einer Stromkonstantstufe 6 aufgeladen. Die Spannung am Kondensator 5 steigt dadurch linear mit der Zeit an. Über einen dem Kondensator vorgeschalteten Schalter 4, der von den Triggerimpulsen des Monoflops 3 gesteuert ist, wird jeweils bei Auftreten einer Herzaktion der Kondensator 5 kurzzeitig entladen. Am Kondensator 5 entsteht so eine periodenproportionale Sägezahnspannung. Über einen Trennverstärker 7 gelangt die Sägezahnspannung auf einen aus Widerstand 8 und Kondensator gebildeten Tiefpaß 10. Dieses RC-Glied bildet jeweils den Mittelwert aus der sägezahnförmigen Eingangsspannung. Der Mittelwert wird bei jeder Herzaktion in einer Sample-&-Hold-Schaltung abgespeichert. Diese Sample-&-Hold-Schaltung besteht im Prinzip aus einem Schalter 9, der von dem ersten Monoflop 2 gesteuert ist, und einem Kondensator 11 als Speicherglied.

Der in der Sample-&-Hold-Schaltung 9, 11 abgespeicherte Spannungspegel gelangt über einen Addierverstärker 12, und einen Widerstand 13 auf den einen Eingang eines ersten Komparators 14. Auf den zweiten Eingang des Komparators 14 gelangt über die Widerstände 15 bis 18 die sägezahnförmige Spannung des Kondensators 5. Dabei sind die Widerstände 16 bis 18 als Spannungsteiler mit zugeordneten Schaltern 19 bis 21 geschaltet. Abhängig von der Anschaltung der Teilwiderstände 16 bis 18 wird also die Sägezahnspannung in einem vorbestimmbaren Verhältnis abgeschwächt. Wird die abgeschwächte Sägezahnspannung größer als der gespeicherte Mittelwert, gibt der Komparator 14 ein Ausgangssignal auf einen monostabilen Multivibrator 35, der einen Zählimpuls zur Weiterverarbeitung auslöst.

Durch Anschaltung der Widerstände 16 bis 18 läßt sich also die Sägezahnspannung justieren. Es kann so bestimmt werden, nach welchem Multiplikationsfaktor $k > 1$ des mittleren RR-Abstandes der Komparator 14 umschaltet. Beispielsweise sind die Spannungsteiler so gewählt, daß die Faktoren $k = 1,6$; $2,6$ oder $3,6$ sind. Der durch die Spannungsteiler 15 bis 18 bestimmte Multiplikationsfaktor $k$ ist dabei unabhängig von der Herzfrequenz.

In einem zweiten Komparator 22 wird die sägezahnförmige Spannung des Kondensators 5 mit einem Festspannungswert, der durch aus Widerständen 23 und 24 gebildeten Spannungsteiler bestimmt ist, verglichen. Überschreitet die Sägezahnspannung den dadurch vorgewählten Spannungswert, so schaltet der Komparator 22 um, wobei über eine nachgeschaltete Diode 25 der Eingang des ersten Komparators 14 auf negatives Potential gelegt wird und der Komparator 14 ein Schaltsignal an Monoflop 35 gibt, so daß ein Zählimpuls ausgelöst wird. Mit einer derartigen Maßnahme ist erreicht, daß nach Ablauf eines maximalen Zeitintervalls nach einer letzten Herzaktion ein Zählimpuls für eine Asystolie oder "missed beat" ausgelöst wird. Ein solches Zeitintervall beträgt beispielsweise 2 s, da aus medizinischen Gründen ein RR-Abstand größer 2 s auf jedenfall als pathologisch zu werten ist.

In einem dritten Komparator 26 wird nun die Mittelwertspannung der Sample-&-Hold-Schaltung 9, 11 mit dem Spitzenwert der sägezahnförmigen Spannung vom Kondensator 5 verglichen. Der Spitzenwert dieser Sägezahnspannung wird über zwei aus den Kondensatoren 30 und 32 mit zugeordneten Schaltern 29 und 31 gebildeten Sample-&-Hold-Schaltungen abgespeichert. Die Schalter 29 und 31 werden dabei jeweils vom ersten und zweiten Monoflop 2 und 3 gesteuert. Diese abgespeicherten Werte gelangen über den aus den Widerständen 33 und 34 gebildeten Spannungsteiler auf den zweiten Eingang des Komparators 26. Ein Ausgangssignal des Komparators 26 bewirkt ein Umschalten eines einem Verstärker 27 nachgeschalteten Schalters 28. Auf den dem ersten Komparator 14 vorgeschalteten Addierverstärker 12 wird bei umgeschaltetem Schalter 28 eine zusätzliche Vorspannung des Verstärkers 27 gelegt. Diese Vorspannung entspricht der Zeitdifferenz von mittlerer Herzperiode zur Zeit einer vorzeitig einfallenden Herzaktion. Damit wird die Ausgangsspannung des Addierverstärkers 12 genau so weit angehoben, wie es der Vorzeitigkeit der Herzaktion entspricht. In diesem Fall führt also eine kompensatorische Pause nach einer vorzeitig aufgetretenen Extrasystole nicht zu einem Zählimpuls.

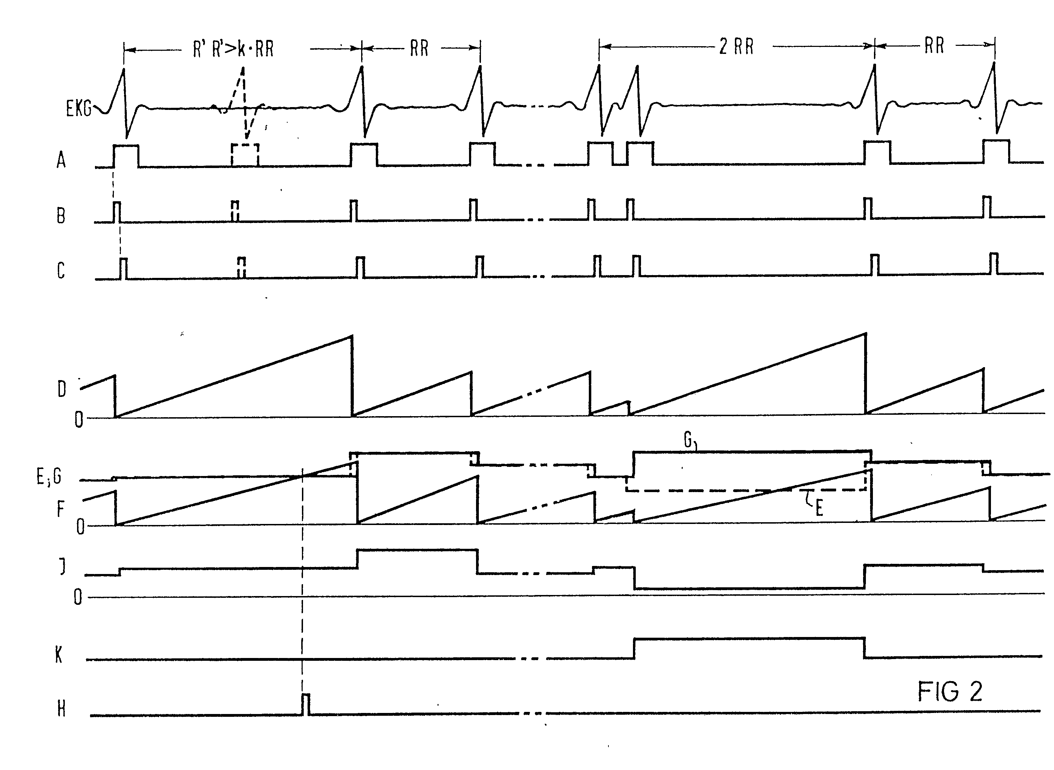

In der Fig. 2 ist in der ersten Zeile ein schematisches EKG angedeutet, aus dem mittels eines R-Zacken-Diskriminators 1 Rechteckimpulse und mittels Monoflops 2 und 3 zeitlich versetzte Triggerimpulse abgeleitet werden. Die Zeile D zeigt als Analogsignal die Sägezahnspannung des Kondensators 5. In der nachfolgenden Zeile sind die gespeicherten Mittelwertspannungen am Punkt E, also vor dem Verstärker 12, und am Punkt G, also nach dem Verstärker 12, dargestellt. Wenn vom Komparator 26 kein Ausgangssignal gegeben wird, verlaufen diese Signalspannungen E und G gleich. Bei Ausfallen eines Ereignisses im EKG - wie in der ersten Zeile symbolisch angedeutet - schneidet die justierte Sägezahnspannung F die Signalspannung G. Zu diesem

0017966

Zeitpunkt wird vom Komparator 14 ein Ausgangssignal erzeugt und damit ein Zählimpuls getriggert. An den zugeordneten Anzeigegeräten wird eine Asystolie bzw. "missed beat" angezeigt.

Im Normalfall würde nun bei einer kompensatorischen Pause nach zwei Doppelsignalen ebenfalls ein Zählsignal getriggert. Durch Zuschalten des Signals des Addierverstärkers 27 durch den Komparator 26 entsprechend Zeile K wird aber in diesem Fall das Signal G gegenüber E angehoben. D.h., zwischen Sägezahnspannung F und Signal G gibt es keinen Schnittpunkt, so daß vom Komparator 14 kein Ausgangssignal erzeugt und damit kein Zählimpuls getriggert wird. Die kompensatorische Pause wird also vor der Weiterverarbeitung eliminiert und nicht als Asystolie oder "missed beat" angezeigt.

Das erfindungsgemäße Verfahren und die zugehörige Vorrichtung wurden speziell für die Auswertung von EKG-Signalfolgen beschrieben. Natürlich sind auch andere periodische Signalfolgen, beispielsweise EEG, Blutdruck oder Atemsignale, entsprechend auswertbar.

Patentansprüche

1. Verfahren zur Auswertung von im wesentlichen periodischen Signalfolgen, insbesondere von physiologischen Signalen, wie EKG od.dgl., d a d u r c h  g e - k e n n z e i c h n e t , daß zur Erkennung und Anzeige von in der im wesentlichen periodischen Signalfolge entsprechend mittlerer Signalfrequenz fehlenden Einzelereignissen eine justierbare, der Signalperiodendauer proportionale Meßspannung mit dem Mittelwert aus der in der vorangehenden Signalperiode aufsummierten Meßspannung verglichen wird, wobei bei Überschreiten des Vergleichsspannungswertes ein Zählimpuls ausgelöst wird.

2. Verfahren nach Anspruch 1, d a d u r c h  g e - k e n n z e i c h n e t , daß durch Vergleich der Meßspannung mit einer vorwählbaren Konstantspannung bei Überschreiten eines durch die Konstantspannung vorgegebenen festen Zeitwertes der Zählimpuls ausgelöst wird.

3. Verfahren nach Anspruch 1 und 2, d a d u r c h  g e k e n n z e i c h n e t , daß Ereignispausen, die lediglich aufgrund eines vorzeitig auftretenden signifikanten Ereignisses verursacht werden, durch Vergleich der Meßspannung mit dem um den Grad der Vorzeitigkeit des erfolgten Signalereignisses entsprechend angehobenen Mittelwert der Vergleichsspannung erkannt werden, wobei solche Ereignispausen nicht zum Auslösen eines Zählimpulses führen.

4. Vorrichtung zur Durchführung des Verfahrens nach Anspruch 1 oder nach Anspruch 1 bis 3, mit einer Schaltungsanordnung zur Auswertung der im wesentlichen peri-

0017966

- 2 -     VPA 79 P 5036 EUR

odischen Signalfolgen hinsichtlich Signalperiode bzw. -frequenz, **d a d u r c h   g e k e n n z e i c h - n e t ,** daß die Schaltungsanordnung einen Meßspannungssummierer (5) für eine periodenproportionale Meßspannung, einen Mittelwertbildner (8, 10), einen Meßspannungsspeicher (9, 11) und wenigstens einen ersten Komparator (14) zum Vergleich des im Meßspannungsspeicher abgespeicherten Mittelwerts der vorangehenden Signalperiode mit der periodenproportionalen Meßspannung aufweist, wobei speziell die Amplitude der periodenproportionalen Meßspannung justierbar ist.

5. Vorrichtung nach Anspruch 4, **d a d u r c h   g e - k e n n z e i c h n e t ,** daß der Meßspannungssummierer in an sich bekannter Weise ein von einer Konstantstromquelle (6) aufladbarer Kondensator (5) ist, der von einer Einheit zur Erkennung von signifikanten Signalereignissen (1) mit nachgeschaltetem Impulsgeber (2, 3) jeweils bei Ablauf der Signalperiode (RR) entladen wird.

6. Vorrichtung nach Anspruch 5, **d a d u r c h   g e - k e n n z e i c h n e t ,** daß der Mittelwertbildner ein aus Widerstand (8) und Kondensator (10) gebildeter Tiefpaß ist, von dem über einen steuerbaren Schalter (9) der Mittelwert auf einen weiteren Kondensator (11) als Meßwertspeicher gebbar ist.

7. Vorrichtung nach Anspruch 4, **d a d u r c h   g e - k e n n z e i c h e t ,** daß die periodenproportionale Meßspannung über eine Mehrzahl angeschalteter Spannungsteiler (15 bis 18) mit zugeordneten Schaltern (19 bis 21) in einem vorwählbaren Verhältnis absenkbar ist.

8. Vorrichtung nach Anspruch 4, d a d u r c h
g e k e n n z e i c h e t , daß der Schaltungsanordnung ein zweiter Komparator (22) zugeordnet ist,
in welchem zweiten Komparator (22) die periodenproportionale Meßspannung mit einer Konstantspannung verglichen wird, wobei das Signal des zweiten Komparators
(22) das Vergleichsspannungssignal des ersten Komparators (14) umpolt, so daß der erste Komparator (14)
durchschaltet und von der nachgeschalteten Schaltstufe
(35) ein Zählimpuls erzeugt wird.

9. Vorrichtung nach Anspruch 4, d a d u r c h
g e k e n n z e i c h n e t , daß der Schaltungsanordnung ein dritter Komparator (26) zugeordnet ist,
in welchem dritten Komparator (26) der justierbare
Spitzenwert der periodenproportionalen Meßspannung
mit dem gespeicherten Mittelwert verglichen wird, wobei bei Unterschreiten eines vorgebbaren Wertes des
ersten Komparators (14), des dritten Komparators (26)
eine Zusatzspannung zum Vergleichsspannungswert anschaltet.

10. Vorrichtung nach Anspruch 9, d a d u r c h
g e k e n n z e i c h n e t , daß der Spitzenwert
des Ladekondensators (5) mittels Sample-&-Hold-Schaltungen (29, 30, 31, 32) abspeicherbar und in einem
nachgeschalteten Spannungsteiler (33, 34) nach vorgebbarem Verhältnis teilbar ist.

11. Vorrichtung nach Anspruch 9, d a d u r c h
g e k e n n z e i c h n e t , daß die Zusatzspannung
zur Anhebung des Vergleichsspannungssignals des ersten
Komparators (14) über einen dem ersten Komparator (14)
vorgeschalteten Addierverstärker (12) aufaddierbar ist.

12. Vorrichtung nach Anspruch 9 bis 11, d a d u r c h
g e k e n n z e i c h n e t , daß die Zusatzspannung
von dem in der Sample-&-Hold-Schaltung (31, 32) gespeicherten Spitzenwert des Ladekondensators (5) ableitbar ist.

13. Vorrichtung nach Anspruch 9 bis 12, d a d u r c h
g e k e n n z e i c h n e t , daß die Zusatzspannung
in einem Verstärker (27) aus dem Mittelwertsignal und
dem Spitzenwertsignal entsprechend der Vorzeitigkeit
des signifikanten Ereignisses gebildet wird.

FIG 1

79 P 5036 1/2

0017966

0017966

79 P 5036    2/2

FIG 2

## EINSCHLÄGIGE DOKUMENTE

KLASSIFIKATION DER ANMELDUNG

| Kategorie | Kennzeichnung des Dokuments mit Angabe, soweit erforderlich, der maßgeblichen Teile | betrifft Anspruch |
|---|---|---|
| | DE - B - 2 713 945 (SIEMENS AG) <br> * Spalte 3, Zeile 64 bis Spalte 5, Zeile 24; Spalte 7, Zeile 4 bis Spalte 8, Zeile 65; Abbildungen 1,2 * <br><br> -- <br><br> MEDICAL AND BIOLOGICAL ENGINEERING Band 11, Nr. 2. März 1973, Stevenage, GB, A. SANDMAN et al.: "Analogue preprocessor for the measurement by a digital computer of RR intervals and R wave width", Seiten 191-200 <br> * Seiten 191-200 * <br><br> -- <br><br> US - A - 3 861 387 (R.D. LAWHORN et al.) <br> * Zusammenfassung; Spalte 6, Zeile 33 bis Spalte 8, Zeile 22; Abbildungen 1A-3D * <br><br> -- <br><br> DE - A - 2 352 692 (BRUKER-PHYSIK AG) <br> * Seite 2, Zeile 19 bis Seite 4, Zeile 6; Seite 6, Zeilen 9-17; Seite 10, Zeilen 1-10; Seite 14, Zeilen 10-21; Seite 23, Zeilen 14-20; Seite 25, Zeile 27 bis Seite 27, Zeile 23; Seite 29, Zeile 5 bis Seite 32 Zeile 4; Abbildungen 1-4 * <br><br> -- <br><br> US - A - 3 699 949 (J.F. O'HANLON et al.) <br> ./. | 1,4 <br><br><br><br><br><br> 1 <br><br><br><br><br><br><br><br><br><br><br> 1,5,10 <br><br><br><br><br><br> 1,4,6 <br><br><br><br><br><br><br><br><br><br><br> 1,4 |

A 61 B 5/04

RECHERCHIERTE SACHGEBIETE

A 61 B 5/04
5/02

KATEGORIE DER GENANNTEN DOKUMENTE

X von besonderer Bedeutung

A technologischer Hintergrund

O nichtschriftliche Offenbarung

P Zwischenliteratur

T der Erfindung zugrunde liegende Theorien oder Grundsätze

E kollidierende Anmeldung

D in der Anmeldung angeführtes Dokument

L aus anderen Gründen angeführtes Dokument

& Mitglied der gleichen Patentfamilie, übereinstimmendes Dokument

Der vorliegende Recherchenbericht wurde für alle Patentansprüche erstellt

| Recherchenort. | Abschlußdatum der Recherche | Prüfer |
|---|---|---|
| Den Haag | 01-08-1980 | RIEB |

EPA form 1503.1 06.78

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | * Zusammenfassung; Spalte 2, Zeile 36 bis Spalte 3, Zeile 67; Abbildungen 1-3 * | |
| | FR - A - 2 267 732 (AGENCE NATIO- NALE DE VALORISATION DE LA RECHER- CHE) <br> * Seite 3, Zeilen 4-12; Seite 4, Zeile 14 bis Seite 8, Zeile 11; Abbildungen 1-3 * | 1,2 |
| | US - A - 4 018 219 (G. HOJAIBAN/ AMERICAN HOME PRODUCTS CORP.) <br> * Zusammenfassung; Spalte 1, Zeile 56 bis Spalte 2, Zeile 45; Spalte 3, Zeile 41 bis Spalte 6, Zeile 2; Abbil- dungen 1,2 * | 1,4-6 <br> 10 |
| P | GB - A - 2 021 778 (C.M. IN- DUSTRIES) <br> * Zusammenfassung; Seite 1, Zeilen 26-53; Seite 1, Zeile 103 bis Seite 2, Zeile 46; Abbildungen 1,2 * | 1,4 |

KLASSIFIKATION DER ANMELDUNG (Int.Cl.)

RECHERCHIERTE SACHGEBIETE (Int. Cl.)